**Europäisches Patentamt**

**European Patent Office**

**Office européen des brevets**

(11) Veröffentlichungsnummer : **0 037 151**
**B1**

(19)

(12)
# EUROPÄISCHE PATENTSCHRIFT

(45) Veröffentlichungstag der Patentschrift :
**13.06.84**

(51) Int. Cl.³ : **A 61 B 6/02, G 01 N 23/08,**
**G 06 F 15/20, G 01 T 1/161**

(21) Anmeldenummer : **81200332.5**

(22) Anmeldetag : **25.03.81**

(54) Computer-Tomographiegerät.

(30) Priorität : **01.04.80 DE 3012648**

(43) Veröffentlichungstag der Anmeldung :
**07.10.81 Patentblatt 81/40**

(45) Bekanntmachung des Hinweises auf die Patenterteilung : **13.06.84 Patentblatt 84/24**

(84) Benannte Vertragsstaaten :
**DE FR GB IT SE**

(56) Entgegenhaltungen :
**WO-A-81 /004 57**
**DE-A- 2 513 137**
**DE-A- 2 625 312**
**DE-A- 2 642 741**
**DE-A- 2 719 856**
**DE-A- 2 754 361**
**FR-A- 2 422 382**

(73) Patentinhaber : **Philips Patentverwaltung GmbH**
**Billstrasse 80**
**D-2000 Hamburg 28 (DE)**
**DE**
**N.V. Philips' Gloeilampenfabrieken**
**Groenewoudseweg 1**
**NL-5621 BA Eindhoven (NL)**
**FR GB IT SE**

(72) Erfinder : **Kowalski, Günter**
**Fasanenweg 8a**
**D-2080 Pinneberg (DE)**

(74) Vertreter : **Hartmann, Heinrich, Dipl.-Ing. et al**
**Philips Patentverwaltung GmbH Billstrasse 80 Post-**
**fach 10 51 49**
**D-2000 Hamburg 28 (DE)**

**Beschreibung**

Die Erfindung betrifft eine Vorrichtung zur Ermittlung der Absorptionsverteilung von Strahlung in einer Ebene eines Untersuchungsbereichs eines Körpers, mit einer um den Körper bewegbaren Strahlenquelle zur Aussendung eines in der Ebene verlaufenden fächerförmigen, den Untersuchungsbereich in unterschiedlichen Richtungen vollständig durchsetzenden Strahlenbündels, und mit einer einzelne Detektoren enthaltenden Detektorreihe zur Erzeugung von Detektorausgangssignalen, die ein Maß für die Absorption der Strahlung sind, und die verstärkt und über Analog-Digital-Wandler einer elektronischen Einheit zur Erzeugung und Darstellung der Absorptionsverteilung aus den Detektorausgangssignalen zugeleitet werden.

Derartige Vorrichtungen (Computer-Tomographiegeräte) sind allgemein bekannt, beispielsweise aus der DE-OS 26 25 312. Jedes Ausgangssignal eines einzelnen Detektors wird dabei jeweils einem Verstärker zugeführt und gelangt von dort über je eine Integrationsschaltung und einen Analog-Digital-Umsetzer zu einem elektronischen Rechner, der aus den so gewonnenen Meßwerten die Absorptionsverteilung der durchstrahlten Körperebene rekonstruiert. Dabei ist es u. a. erforderlich, die Meßwerte nach einer Normierung und Logarithmierung miteinander durch eine Faltung zu verknüpfen, was ebenfalls in dem elektronischen Rechner geschieht.

Insbesondere die Genauigkeitsunterschiede benachbarter Verstärker führen jedoch zu Artefakten (z. B. Ringmuster) in der rekonstruierten Körperschicht. Damit derartige Artefakte nicht zu stark hervortreten, sind aufwendige und mit hoher Genauigkeit arbeitende elektronische Einrichtungen zur Verarbeitung der Detektorausgangssignale erforderlich.

Aufgabe der Erfindung ist es, eine Vorrichtung zur Ermittlung der Absorptionsverteilung von Strahlung in einem ebenen Untersuchungsbereich eines Körpers anzugeben, bei der weniger genau arbeitende elektronische Einrichtungen zur Verarbeitung der Detektorausgangssignale eingesetzt werden können, ohne daß dadurch Artefakte im rekonstruierten bild entstehen.

Diese Aufgabe wird gemäß der Erfindung dadurch gelöst, daß wenigstens in einem Teilbereich der Detektorreihe Mittel vorhanden sind, die von jeweils zwei benachbarten Detektoren einen der Differenz der unverstärkten Detektorausgangssignale proportionalen Differenzmeßwert bilden, daß wenigstens ein Detektor dieses Bereiches zusätzlich mit weiteren Mitteln verbunden ist, die einen seinem Detektorausgangssignal proportionalen absoluten Meßwert erzeugen, und daß Additionsmittel zur Addition der Differenzmeßwerte zum absoluten Meßwert vorhanden sind.

Ist ein einem Detektorausgangssignal proportionaler absoluter Meßwert M1 bekannt, beispielsweise durch direkte Messung mittels eines äußeren oder in der Mitte liegenden Detektors der Detektorreihe, und wird von den unverstärkten Detektorausgangssignalen jeweils zweier benachbarter Detektoren direkt die Differenz gebildet, z. B. indem die Ausgänge von jeweils zwei benachbarten Detektoren mit einem als Subtrahierglied arbeitenden Differenzverstärker 18a, b, c... verbunden sind, so daß auf diese Weise Differenzmeßwerte $(M2 - M1)$, $(M3 - M2)$, ... erzeugt werden, so können durch fortlaufende Addition der Differenzmeßwerte zu dem absoluten Meßwert weitere, den restlichen Detektoren zugeordnete absolute Meßwerte $\overline{M2} = M1 + (M2 - M1)$, $\overline{M3} = M1 + (M2 - M1) + (M3 - M2)$, usw. ermittelt werden, die denen entsprechen, die bei der direkten Messung der absoluten Meßwerte jedes einzelnen Detektors erhalten worden wären. Die durch die Addition gebildeten absoluten Meßwerte $\overline{M2}$, $\overline{M3}$, ... sind dabei zwar mit dem Fehler behaftet, der bei der Messung des ersten absoluten Meßwertes $M1 = \overline{M1}$ auftritt. Dies ist jedoch unerheblich, da er bei allen Meßwerten auftritt und daher nicht zur Artefaktbildung beiträgt. Diejenigen Fehler aber, die aufgrund der direkten Differenzbildung der Detektorausgangssignale auftreten, und die von dem Fehler des Verstärkungsfaktors der Differenzverstärker und der Differenz der Detektorausgangssignale abhängen, sind relativ klein und können sich zudem bei der Addition der Differenzmeßwerte herausmitteln.

Durch direkte Differenzbildung der Detektorausgangssignale wird daher erreicht, daß bei gleichen Anforderungen an die Artefaktfreiheit der rekonstruierten Absorptionsverteilung die die Detektorausgangssignale verstärkenden Verstärker weniger genau zu sein brauchen. Die absoluten Meßwerte $\overline{M1}$, $\overline{M2}$, $\overline{M3}$, ... können dann über jeweils einen oder über einen für alle Meßwerte gemeinsamen Analog-Digital-Wandler dem elektronischen Rechner zugeführt werden.

Nach einer vorteilhaften Weiterbildung der Erfindung sind die Additionsmittel mit den Ausgängen der Analog-Digital-Wandler zur Addition der digitalisierten Differenzmeßwerte $((M2 - M1)$, $(M3 - M2)$, ...) zum digitalisierten absoluten Meßwert M1 verbunden.

Die Differenzmeßwerte $(M2 - M1)$, $(M3 - M2)$, ... und der absolute Meßwert M1 werden also vor ihrer Addition einem Analog-Digital-Wandler zugeführt. Hierdurch wird erreicht, daß die Analog-Digital-Wandler im Vergleich zu denen der bekannten Anordnung eine geringere Auflösung, d. h. eine kleinere Bitzahl besitzen können.

Die Zeichnung stellt Ausführungsbeispiele der Erfindung dar. Es zeigen :

Figur 1 ein bekanntes Computer-Tomographiegerät,

Figur 2 eine Detektoranordnung mit bekannter Signalverarbeitung,

Figur 3 eine Detektoranordnung mit Mitteln zur Bildung der Differenz der Detektorausgangssignale,

Figur 4 eine Skizze zur Erläuterung der Auflösungsvermögens bei Analog-Digital-Wandlern,

Figur 5 eine Detektoranordnung aus Ionisationskammern zur direkten Bildung der Differenz der Detektorausgangssignale, und

Figur 6 eine weitere Detektoranordnung aus Photodioden.

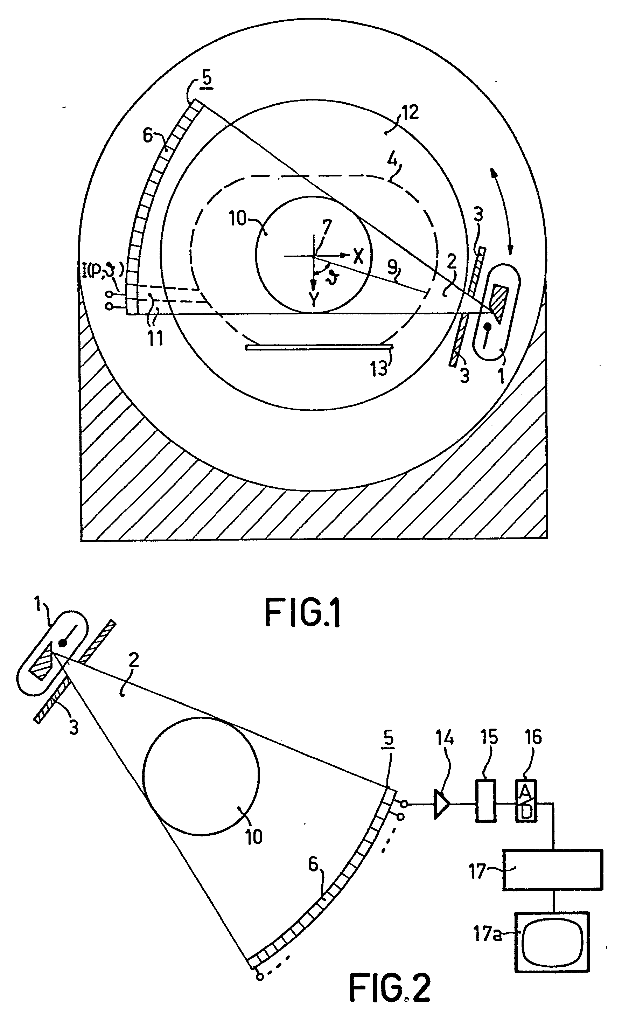

Fig. 1 zeigt einen Schnitt durch ein schematisch dargestelltes Röntgentomographiegerät, welches aus einer Strahlenquelle 1 zum Aussenden eines fächerförmigen Röntgenstrahlenbündels 2 besteht, das in der Schnittebene (Zeichenebene), die die Untersuchungsebene darstellt, verläuft, und, das mittels einer Blende 3 begrenzt ist. Das Röntgenstrahlenbündel 2 durchdringt einen zu untersuchenden Körper 4 und trifft auf eine Detektorreihe 5, welche aus einzelnen in der Untersuchungsebene nebeneinanderliegenden Strahlungsdetektoren 6 besteht. Das System Strahlenquelle 1 — Detektorreihe 5 ist um eine Zentralachse 7, die senkrecht zur Untersuchungsebene verläuft, drehbar angeordnet, wobei seine Stellung relativ zu einem in der Untersuchungsebene liegenden, rechtwinkligen Koordinatensystem X, Y durch einen Drehwinkel θ, den der Zentralstrahl 9 des fächerförmigen Strahlenbündels 2 mit der Y-Achse einschließt, angegeben wird, während p den Abstand eines Strahlenweges vom Koordinatenursprung angibt. Der Ursprung des Koordinatensystems X, Y, durch den die Zentralachse 7 hindurchläuft, ist gleichzeitig Zentrum des Untersuchungsbereichs 10 des Röntgentomographiegerätes. Dies ist der in der Untersuchungsebene liegend Bereich, der unter jedem Drehwinkel θ vollständig durchstrahlt wird, wobei die Breite der Strahlenwege 11 durch die Breite der Detektoren 6 bestimmt ist. Die Detektorausgangssignale sind mit I (p, θ) bezeichnet und entsprechen der Absorption der Strahlung entlang der Strahlenwege 11.

Zur Lagerung des zu untersuchenden Körpers 4 (gestrichelt gezeichnet), der in einem den Untersuchungsbereich 10 konzentrisch umgebenden Lagerungsbereich 12 liegt, ist ein senkrecht zur Untersuchungsebene verstellbarer Patientisch 13 vorgesehen. Seine mechanische Halterung ist der Übersicht wegen nicht dargestellt. Durch eine Veränderung der Lage des Körpers 4 innerhalb des Lagerungsbereichs 12 kann zudem erreicht werden; daß der Untersuchungsbereich 10, der in seiner Größe durch Verstellen der Blende 3 veränderbar ist, verschiedene zu diagnostizierende Bereiche innerhalb des Körpers 4 überdeckt. Hierzu muß natürlich zwischen dem Körper 4 und dem Lagerungsbereich 12 genügend Zwischenraum sein.

In Fig. 2 ist dargestellt, wie die Detektorausgangssignale I (p, θ) in bekannter Weise elektronisch verarbeitet werden. Dabei sind die Ausgänge der einzelnen Detektoren 6 jeweils mit einem Verstärker 14 verbunden, über den die Detektorausgangssignale zu einem Integrationsglied 15 und zu einem Analog-Digital-Wandler 16 gelangen, so daß am Ausgang des Analog-Digital-Wandlers absolute, digitalisierte Meßwerte vorliegen. Von dort werden die Meßwerte an einen elektronischen Rechner 17 zur Rekonstruktion der Absorptionsverteilung weitergeleitet, der z. B. mit einem Monitor 17a zur Darstellung der Absorptionsverteilung verbunden ist. An den Ausgang eines jeden Detektors 6 ist somit eine solche Reihe von elektronischen Einheiten (14-16) geschaltet, wobei der Verstärker 14 und has Integrationsglied 15 auch zu einer Einheit zusammengefaßt sein können.

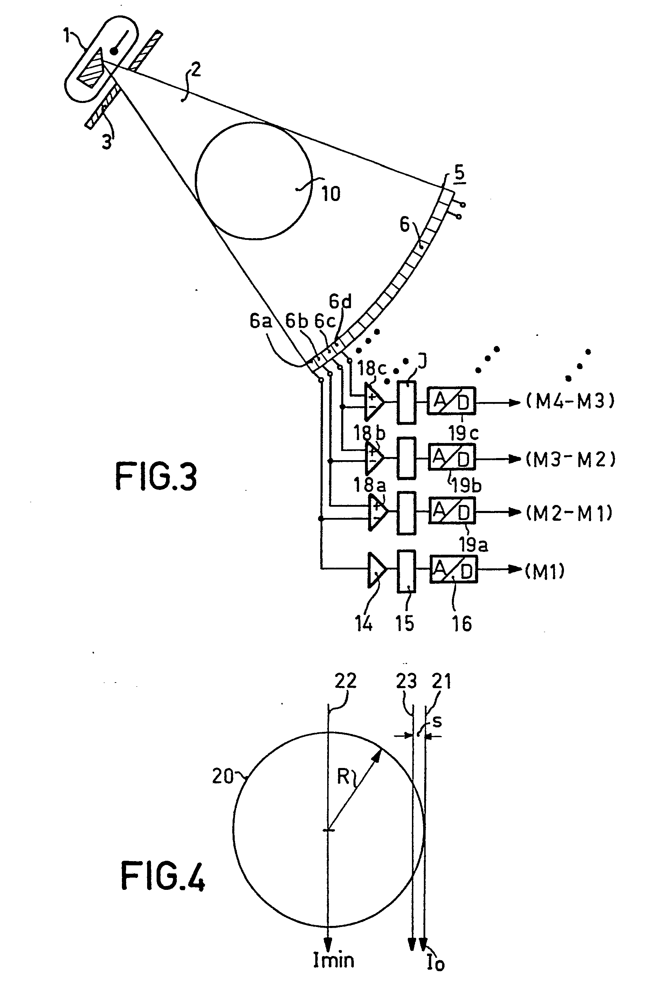

Sollen nun bei der Verarbeitung der Detektorausgangssignale weniger genau arbeitende Verstärker und Analog-Digital-Wandler eingesetzt werden, ohne daß dadurch Artefakte im zu rekonstruierenden Bild entstehen, so wird gemäß der Erfindung eine Anordnung nach Fig. 3 vorgeschlagen, bei der die Differenz der unverstärkten Detektorausgangssignale I (p, θ) von jeweils zwei benachbarten Detektoren 6a, b ; 6b, c usw. direkt gebildet wird. Hierzu werden z. B. Verstärker 18a, b, c, ... in Differenzschaltung benutzt (Differenzverstärker). Dies bedeutet im allgemeinen keinen zusätzlichen Aufwand, da ein moderner Verstärker meistens einen Differenzeingang besitzt, von denen sonst ein Eingang auf « Null » liegt. Es ist für den Erfindungsgedanken unerheblich, ob die Verstärker 18a-c usw. lineare Verstärker oder auch Integratoren sind. Wesentlich ist die Differenzbildung der Detektorausgangssignale I (p, θ) am Eingang der Verstärker.

Die Ausgangssignale der Differenzverstärker 18a-c... werden über Integratoren I zu Analog-Digital-Wandlern 19a-c... geleitet, an deren Ausgängen dann digitalisierte Differenzmeßwerte (M2 − M1), (M3 − M2) usw. erscheinen. Zusätzlich wird aber bei einem oder mehreren Detektoren der Detektorreihe 5, z. B. dem äußeren Detektor 6a, ein dem Detektorausgangssignal proportionaler absoluter Meßwert M1 gemessen, wozu dieser Detektor 6a zusätzlich mit einem Verstärker 14, einem Integrator 15 und einem Analog-Digital-Wandler 16 verbunden ist, an dessen Ausgang dann ein dem Detektorausgangssignal entsprechender digitalisierter absoluter Meßwert M1 erscheint.

Die Ermittlung von absoluten Meßwerten $\overline{M2}$, $\overline{M3}$... für die restlichen Detektoren 6a, c, d usw., die im Rechner 17 vor der Faltung normiert und logarithmiert werden, erfolgt dann so, daß die jeweiligen digitalisierten Differenzmeßwerte (M2 − M1), (M3 − M2), ... zum absoluten digitalisierten Meßwert $\underline{M1}$ (M1 − $\overline{M1}$) addiert werden. Man erhält z. B. $\overline{M2}$ für den Detektor 6b gemäß der Formel $\overline{M2}$ = M1 + (M2 − M1), wenn der Detektor 6b der zweite Detektor in der Detektorreihe 5 ist, und (M2 − M1) der Differenzmeßwert ist, der aus den Detektorausgangssignalen des zweiten und des ersten Detektors 6b a abgeleitet wurde.

Für den dritten Detektor 6c der Detektorreihe 5 eirgibt sich der absolute Meßwert $\overline{M3}$ zu

$$\overline{M3} = M1 + (M2 − M1) + (M3 − M2),$$

wobei (M3 − M2) der Differenzmeßwert ist, der aus den Detektorausgangssignalen des dritten und zweiten Detektors 6c, 6b abgeleitet wurde. Für die weiteren absoluten Meßwerte $\overline{M4}$... usw. gilt entsprechendes.

Der Fehler, der dem Meßwert M1 anhaftet, erscheint natürlich bei allen weiteren errechneten absoluten Meßwerten. Er trägt aber nicht zur Artefaktbildung bei, da alle Meßwerte mit ihm beaufschlagt werden. Die bei der Differenzbildung der Detektorausgangssignale entstandenen Fehler sind dagegen aber sehr klein und mitteln sich bei der Berechnung der absoluten Meßwerte aus den Differenzmeßwerten in der Regel heraus. Aus diesem Grund können in ihrer Genauigkeit verminderte Verstärker 14, 18a, b, c (und Integratoren 15, I) verwendet werden.

Da ferner die Differenzmeßwerte (M2 − M1), (M3 − M2), ... bzw. der absolute Meßwert M1 erst nach Durchlaufen der Analog-Digital-Wandler 16, 19a-c ... miteinander addiert werden, können weiterhin die Analog-Digital-Wandler eine geringere Auflösung, d. h. eine kleinere Bitzahl besitzen als diejenigen der bekannten Anordnung.

Dies sei anhand der Fig. 4 nochmals erläutert, die ein Objekt 20 mit einem Radius R = 15 cm und einer Absorption von $\mu = 0.2$ cm$^{-1}$ (etwa Wasser) darstellt. Die Intensität außerhalb des Objektes sei $I_o$, gemessen durch den Strahl 21. Die minimale Intensität ist $I_{min}$, gemessen durch Strahl 22. Bei den gegebenen Werten ist

$$I_{min} = I_o \, e^{-2/\mu R} = I_o \, e^{-6} = 0.002 \, 48 \, I_o.$$

Soll dieser Wert auf etwa 1‰ genau gemessen werden, so darf das Meßsignal der bekannten Anordnung nur einen relativen Fehler (z. B. durch Verstärkerdirft o. ä.) von ca. $2{,}48 \times 10^{-6}$ besitzen. Dies erfordert sehr genaue Verstärker und einen Analog-Digital-Wandler von ca. 20 bit Auflösung.

Wird die Differenz der unverstärkten Detektorausgangssignale gebildet, so können die relativen Fehler bezüglich der Differenzmeßwerte (M2 − M1), (M3 − M2), ... größer sein, so daß Analog-Digital-Wandler mit geringerer Bitzahl eingesetzt werden können.

Die größte Differenz $I_{max}$ der Meßwerte tritt am Objektrand auf, wenn ein Strahl gerade am Objekt vorbeigeht, Strahl 21, und der benachbarte Strahl 23, versetzt um eine Detektorbreite s, das Objekt 20 schneidet. Die Weglänge durch das Objekt 20 ist

$$\sigma = 2 \sqrt{R^2 - (R - s)^2} = 2 \sqrt{s \, (2R - s)}$$

Wird das Verhältnis 2 R/s = N bezeichnet, das die Anzahl der Detektorelemente angibt, die das Objekt überstreicht, so folgt

$$\sigma = 4 \, R \, \sqrt{N - 1/N}.$$

Die Intensitätsdifferenz wird dann für N = 200

$$\Delta I_{max} = I_o - I_o \, e^{-\mu\sigma} = I_o(1 - e^{-\mu\sigma}) \approx 0.5 \, I_o$$

In der Praxis ergibt sich jedoch, z. B. durch die verbreiterte Strahlgeometrie, ein wesentlich kleinerer Wert von etwa $\Delta I_{max} \approx 0.2 \, I_o$.

Hieraus ergibt sich ein vergrößerter relativer Fehler (proportional $1/\Delta I_{max}$), so daß mehrere bit für einen Analog-Digital-Wandler eingespart werden können.

Einen ganz wesentlichen Vorteil bringt die vorgeschlagene Methode in der Nähe des Rotationszentrums, da hier $\Delta I_{max}$ stets viel kleiner ist (auch ohne Objekt), so daß weitere bit für einen Analog-Digital-Wandler eingespart werden können, bzw. schlechtere Verstärker hinreichend sind.

Um zu vermeiden, daß die berechneten absoluten Meßwerte $\overline{M1}$, $\overline{M2}$, $\overline{M3}$, ... zu weit von den tatsächlichen Meßwerten abweichen, können von mehreren über die Detektorreihe 5 verteilten Detektoren zusätzlich die absoluten Meßwerte gemessen werden. Ergeben sich dann Unterschiede zwischen einzelnen gemessenen und den mittels der Differenzmeßwerte errechneten absoluten Meßwerten, so wird dieser Fehler möglichst gleichmäßig auf viele Meßwerte verteilt und verliert dadurch an Einfluß. Einzelne absolute Meßwerte können dabei durch weitere Anordnungen gemäß Fig. 2 (Elemente 14-16), die zusätzlich mit den Detektoren 6 verbunden sind, gemessen werden.

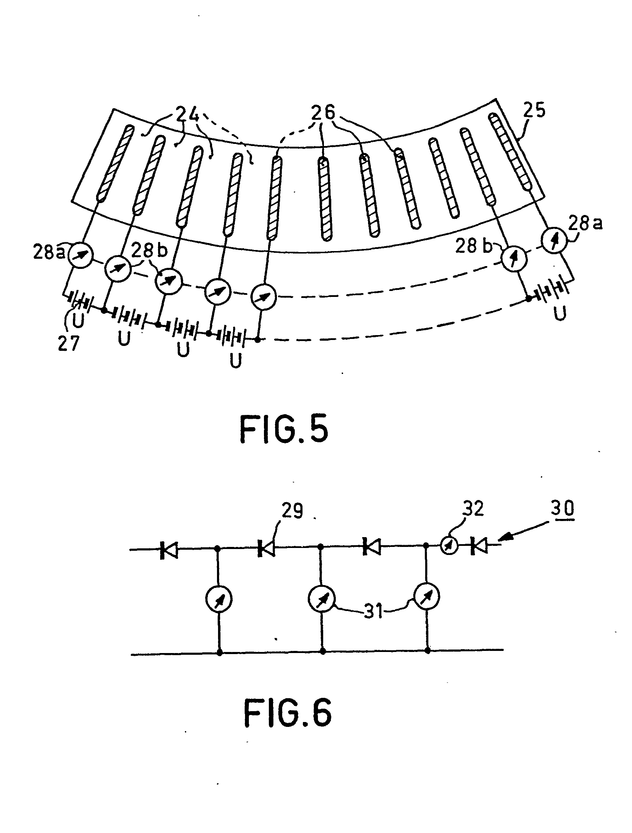

In einer Weiterführung der Erfindung können auch Detektorelemente gebaut werden, die die Differenz der Detektorausgangssignale direkt bilden, um auch noch die Differenzbildung, die sehr genau sein muß, zu ersparen. Ein möglicher Vorschlag ist in Fig. 5 dargestellt. Eine Kette aus Ionisationskammern 24, die eine Detektorreihe 25 bildet, wird mit Spannungen U versorgt. Die Spannung U steigt in einer Richtung der Detektorreihe 25 von der einen zur anderen Elektrodenplatte 26 um gleiche Beträge kaskadenförmig an. Die Elektrodenplatten 26 sind dabei untereinander in gleicher Weise beabstandet. Die notwendige Stromversorgung ist hier nur symbolisch durch Batterien 27 angedeutet, und die Meßgeräte 28a, b messen den Ionisationsstrom. Die Meßgeräte 28a zeigen dann die absoluten Meßwerte an, während die Meßgeräte 28b jeweils die Differenzmeßwerte angeben. Dabei liegen allerdings die Verstärker auf Potentialen, die ein Vielfaches von U betragen, d. h. die Meßwerte müssen z. B. durch optische Koppler ausgekoppelt werden. Ein zu hoher Potentialunterschied läßt sich aber z. B. durch abwechselnde Aktivierung der Spannungsquellen vermeiden.

Ähnliche Anordnungen sind auch möglich bei Detektorreihen, die z. B. Szintillatoren mit optisch angekoppelten Photodioden 29 besitzen, wobei das Kaskadieren wegen der wesentlich kleineren Spannungen erleichtert wird. In Fig. 6 ist eine solche Diodenreihe 30 dargestellt, wobei die Dioden 29 im Kurzschlußbetrieb betrieben werden. Zwischen jeweils zwei Dioden 29 und einem für alle Dioden 29 gemeinsamen Potential liegen Meßgeräte 31 zur Messung der für jeweils zwei Dioden 29 in entgegengesetzter Richtung verlaufenden Ströme, wobei diese Meßgeräte 31 Differenzmeßwerte anzeigen. Mittels des Meßgerätes 32, das nur den Strom durch eine Diode mißt, wird dagegen ein absoluter Meßwert erzeugt.

**Ansprüche**

1. Vorrichtung zur Ermittlung der Absorptionsverteilung von Strahlung in einer Ebene eines Untersuchungsbereichs eines Körpers (10), mit einer um den Körper bewegbaren Strahlenquelle (1) zur Aussendung eines in der Ebene verlaufenden fächerförmigen, den Untersuchungsbereich in unterschiedlichen Richtungen vollständig durchsetzenden Strahlenbündels (2), und mit einer einzelne Detektoren (6a-6d) enthaltenden Detektorreine (5) zur Erzeugung von Detektorausgangssignalen, die ein Maß für die Absorption der Strahlung sind, und die verstärkt und über Analog-Digital-Wandler (19a-19d) elektronischen Einheit zur Erzeugung und Darstellung der Absorptionsverteilung aus den Detektorausgangssignalen zugeleitet werden, dadurch gekennzeichnet, daß wenigstens in einem Teilbereich der Detektorreihe (5) Mittel (18a, b, c, ...) vorhanden sind, die von jeweils zwei benachbarten Detektoren (6a, b, c, ...) einen der Differenz der unverstärkten Detektorausgangssignale proportionalen Differenzmeßwert ((M2 − M1), (M3 − M2), ...) bilden, daß wenigstens ein Detektor (6a) dieses Bereiches zusätzlich mit weiteren Mitteln (14) verbunden ist, die einen seinem Detektorausgangsiganl proportionalen absoluten Meßwert (M1) erzeugen, und daß Additionsmittel zur Addition der Differenzmeßwerte zum absoluten Meßwert vorhanden sind.

2. Vorrichtung nach Anspruch 1, dadurch gekennzeichnet, daß die Additionsmittel mit den Ausgängen der Analog-Digital-Wandler (19a, b, c, ...) zur Addition der digitalisierten Differenzmeßwerte zum digitalisierten absoluten Meßwert verbunden sind.

3. Vorrichtung nach Anspruch 1 oder 2, dadurch gekennzeichnet, daß zur Bildung der Differenzmeßwerte ((M2 − M1), (M3 − M2), ...) aus den Detektorausgangssignalen die Ausgänge von jeweils zwei benachbarten Detektoren (6a, b, ...) mit einem Subtrahierglied (18a, b, c, ...) verbunden sind.

4. Vorrichtung nach Anspruch 3, dadurch gekennzeichnet, daß das Subtrahierglied (18a, b, c, ...) ein Differenzverstärker ist.

5. Vorrichtung nach einem der Ansprüche 3 und 4, dadurch gekennzeichnet, daß die Subtrahierglieder nur für die im zentralen Bereich der Detektorreihe (5) liegenden Detektoren vorgesehen sind.

6. Vorrichtung nach Anspruch 1 oder 2, dadurch gekennzeichnet, daß die Detektorreihe aus einer Reihe von einzelne Detektoren darstellenden Ionisationskammern (24) besteht und durch eine Folge von in gleicher Weise beabstandeter Elektrodenplatten (26) gebildet wird, daß die Spannung an den Elektrodenplatten in einer Richtung der Detektorreihe um gleiche Beträge kaskadenförmig ansteigt, und daß zur Bildung der Differenzmeßwerte aus den Detektorausgangssignalen zwischen den Spannungsabgriffen und den entsprechenden Elektrodenplatten jeweils ein Meßgerät (28) zur Erfassung des gesamten, über die jeweilige Elektrodenplatte fließenden Ionisationsstromes vorgesehen ist.

7. Vorrichtung nach Anspruch 1 oder 2, dadurch gekennzeichnet, daß die einzelnen Detektoren aus auf Röntgenstrahlung ansprechenden Photodioden (29) bestehen, die zur Bildung einer Detektorreihe (30) in gleicher Weise hintereinander geschaltet sind, daß zur Bildung der Differenzmeßwerte zwischen jeweils zwei Dioden und einem für alle Dioden gemeinsamen Potential ein Meßgerät (31) zur Erfassung des Kurzschlußstromes beider Dioden angeordnet ist, und daß wenigstens zwischen einem weiteren Diodenpaar ein weiteres Meßgerät (32) zur Erfassung des durch eine Diode fließenden Stromes liegt.

**Claims**

1. A device for determining the absorption distribution of radiation in a plane of an examination zone of a body (10), comprising a radiation source (1) which is movable about the body and which emits a fan-shaped radiation beam (2) extending in the plane and completely irradiating the examination zone in different directions, and also comprising a detector array (5) which comprises individual detectors (6a-6d) for generating detector output signals which are a measure of the absorption of the radiation, and which are amplified and applied, via analog-to-digital converters (19a-19d), to an electronic unit for generating and displaying the absorption distribution from the detector output signals, characterized in that in at least a part of the detector output signals, characterized in that in at least a part of the detector array (5) there are provided means (18a, b, c ...) which for each pair of adjacent detectors (a, b, c ...) form a difference measurement value ((M2 − M1), (M3 − M2) ...) which is proportional to the difference between the unamplified detector output signals, at least one detector (6a) of this part being additionally connected to further means (14) which form an absolute measurement value (M1) which is proportional to its detector output signal, adding means being provided for adding the difference measurement values to the absolute measurement value.

2. A device as claimed in Claim 1, characterized in that the adding means are connected to the outputs of the analog-to-digital converters (19a, b, c ...) in order to add the digitized difference measurement values to the digitized absolute measurement value.

3. A device as claimed in Claim 1 or 2, characterized in that in order to form the difference measurement values ((M2 − M1), (M3 − M2), ...)

from the detector output signals, the outputs of each pair of adjacent detectors (6a, b, ...) are connected to a subtracting member (18a, b, c, ...).

4. A device as claimed in Claim 3, characterized in that the subtracting member (18a, b, c, ...) is a differential amplifier.

5. A device as claimed in one of the Claims 3 and 4, characterized in that the subtracting members are provided only for the detectors situated in the central part of the detector array (5).

6. A device as claimed in Claim 1 or 2, characterized in that the detector array consists of a row of ionization chambers (24) which represents individual detectors and is formed by a series of uniformly spaced electrode plates (26), the voltage on the electrode plates increasing in a cascade-like manner by equal amounts in one direction along the detector array, between each of the voltage tappings and the corresponding electrode plates there being provided a corresponding measurements apparatus (28) for measuring the ionization current flowing across the relevant electrode plate in order to form the difference measurement values from the detector output signals.

7. A device as claimed in Claim 1 or 2, characterized in that the individual detectors consists of photodiodes (29) which respond to X-rays and which are connected one after another in the same manner in order to form a detector array (30), a measurement apparatus (31) for measuring the short-circuit current of both diodes being connected between each pair of diodes and a potential which is common to all diodes in order to form the difference measurement values, at least one further measurement apparatus (32) for measuring the current flowing through one diode being connected between at least one further diode pair.

**Revendications**

1. Dispositif pour déterminer la répartition de l'absorption du rayonnement dans un plan d'un domaine à examiner d'un corps (10), comportant une source de rayons (1) mobile autour du corps pour émettre un faisceau de rayons (2) en éventail s'étendant dans le plan et traversent complètement le domaine à examiner dans des directions différentes et une rangée de détecteurs (5) contenant des détecteurs individuels (6a-6d) pour produire des signaux de sortie de détecteurs qui sont une mesure de l'absorption du rayonnement et qui sont transmis, à l'état amplifié et par l'intermédiaire de convertisseurs analogiques-numériques (19a-19d), à une unité électronique pour produire et afficher la répartition de l'absorption à partir des signaux de sortie de détecteurs, caractérisé en ce qu'au moins dans une zone partielle de la rangée de détecteurs (5) sont présents des moyens (18a, b, c, ...) qui, à partir chaque fois de deux détecteurs voisins (6a, b, c, ...), forment une valeur de mesure de différence ((M2 − M1), (M3 − M2), ...) proportionnelle à la différence des signaux de sortie de détecteurs non amplifiés, au moins un détecteur (6a) de cette zone est connecté, en outre, à d'autres moyens (14) qui produisent une valeur de mesure absolue (M1) proportionnelle au signal de sortie de ce détecteur et des moyens d'addition sont présents pour additionner les valeurs de mesure de différence et la valeur de mesure absolue.

2. Dispositif suivant la revendication 1, caractérisé en ce que les moyens d'addition sont connectés aux sorties des convertisseurs analogiques-numériques (19a, b, c, ...) pour additionner les valeurs de mesure de différence digitalisées et la valeur de mesure absolue digitalisée.

3. Dispositif suivant la revendication 1 ou 2, caractérisé en ce que, pour former les valeurs de mesure de différence ((M2 − M1), (M3 − M2), ...) à partir des signaux de sortie de détecteurs, les sorties de chaque fois deux détecteurs voisins (6a, b, ...) sont connectées à un élément soustracteur (18a, b, c, ...).

4. Dispositif suivant la revendication 3, caractérisé en ce que l'élément soustracteur (18a, b, c, ...) est un amplificateur de différence.

5. Dispositif suivant l'une des revendications 3 et 4, caractérisé en ce que les éléments soustracteurs ne sont prévus que pour les détecteurs se trouvant dans la zone médiane de la rangée de détecteurs (5).

6. Dispositif suivant la revendication 1 ou 2, caractérisé en ce que la rangée de détecteurs est formée d'une rangée de chambres d'ionisation (24) représentant des détecteurs individuels et par une succession de plaques-électrodes (26) espacées les unes des autres de distances égales, la tension sur les plaques-électrodes augmentant en cascade dans des mesures égales dans un sens de la rangée d'électrodes et, pour former les valeurs de mesure de différence à partir des signaux de sortie de détecteurs entre les prises de tension et les plaques-électrodes correspondantes, un appareil de mesure (28) est chaque fois prévu pour détecter l'ensemble du courant d'ionisation traversant les plaques-électrodes correspondantes.

7. Dispositif suivant la revendication 1 ou 2, caractérisé en ce que les détecteurs individuels sont constitués de photodiodes (29) réagissant aux rayons X, qui sont connectées de la même manière les unes à la suite des autres pour former une rangée de détecteurs (30), un appareil de mesure (31) est prévu pour former les valeurs de mesure de différence chaque fois entre deux diodes et un potentiel commun à toutes les diodes et détecte le courant de court-circuit des deux diodes et un autre appareil de mesure (32) est prévu au moins entre une autre paire de diodes pour détecter le courant traversant une diode.

FIG.1

FIG.2

FIG.3

(M4-M3)

(M3-M2)

(M2-M1)

(M1)

FIG.4

FIG.5

FIG.6